# EUROPÄISCHE PATENTSCHRIFT

(11) **EP 1 786 369 B1**
(45) Veröffentlichungstag und Bekanntmachung des Hinweises auf die Patenterteilung: **06.08.2014**
(21) Anmeldenummer: 05779465.3
(22) Anmeldetag: 20.08.2005
(51) Int. Cl.: A61F 2/46

(54) **CHIRURGISCHES INSTRUMENT**
SURGICAL INSTRUMENT
INSTRUMENT CHIRURGICAL

(30) Priorität: 08.09.2004 DE 102004043995
(43) Veröffentlichungstag der Anmeldung: 23.05.2007
(73) Patentinhaber: Aesculap AG, 78532 Tuttlingen (DE)
(72) Erfinder: FISCHER, Kay, 78532 Tuttlingen (DE); SCHNEID, Susanne, 89233 Neu-Ulm (DE); SCHULTZ, Robert, 78532 Tuttlingen (DE)
(74) Vertreter: Hoeger, Stellrecht & Partner Patentanwälte
(86) Internationale Anmeldenummer: PCT/EP2005/009021
(87) Internationale Veröffentlichungsnummer: WO 2006/027098

(56) Entgegenhaltungen:
- WO-A-00/44321
- WO-A-97/06753
- WO-A-2004/039291

## Beschreibung

Die vorliegende Erfindung betrifft ein chirurgisches Instrument zur Größen- und/oder Höhenbestimmung eines in einen Zwischenwirbelraum zwischen zwei benachbarte Wirbelkörper zweier Wirbel einer menschlichen oder tierischen Wirbelsäule einsetzbaren Zwischenwirbelimplantats, wobei das Zwischenwirbelimplantat ein erstes Anlageelement zur Anlage an eine Gelenkfläche des einen der zwei benachbarten Wirbelkörper und ein am ersten Anlageelement direkt oder indirekt gelagertes zweites Anlageelement zur Anlage an eine Gelenkfläche des anderen der zwei benachbarten Wirbelkörper aufweist, wobei das Instrument ein eine Längsrichtung definierendes Halteteil und mindestens ein Probeimplantat umfaßt, welches ein erstes und ein zweites, jeweils mindestens eine Anlagefläche zur Anlage an einer der Gelenkflächen aufweisendes Probeimplantatanlageelement umfaßt, wobei an einem distalen Ende des Halteteils mindestens ein Anlageelementhalteelement zum lösbaren Verbinden mit dem mindestens einen Probeimplantat vorgesehen ist, wobei das erste und/oder das zweite Probeimplantatanlageelement bezogen auf die Längsrichtung in mindestens zwei definierten, unterschiedlichen Winkelstellungen relativ zu dem mindestens einen Anlageelementhalteelement mit diesem lösbar verbindbar ist und wobei eine Klemmrichtung vorgesehen ist zum klemmenden Sichern einer Verbindung des mindestens einen Anlageelementhalteelements mit dem ersten und/oder dem zweiten Probeimplantatanlageelement in einer der Winkelstellungen.

Zur Behandlung degenerativer Erkrankungen im Bereich der Wirbelsäule wird sehr häufig Bandscheibengewebe operativ entfernt. Anschließend werden benachbarte Bewegungssegmente, das heißt benachbarte Wirbelkörper, zum Beispiel mittels eines Knochenspans und/oder künstlichen Zwischenwirbelimplantaten fusioniert. Eine Alternative zur Fusion benachbarter Wirbelkörper bildet die Implantation von Bandscheibenprothesen, mit denen eine Beweglichkeit benachbarter Wirbelkörper relativ zueinander zumindest teilweise erhalten werden kann.

Eine Bandscheibenprothese dient unter anderem dazu, die ursprüngliche Bandscheibenhöhe wiederherzustellen und die Wirbelsäule wieder zu stabilisieren.

Zur Bestimmung der erforderlichen Größe beziehungsweise Höhe des Zwischenwirbelimplantats sind verschiedene Instrumente oder Probeimplantate bekannt, welche zur Bestimmung von Größe und Höhe in den ausgeräumten Zwischenwirbelraum eingeführt werden. In Abhängigkeit der mit dem Instrument beziehungsweise mit dem Probeimplantat bestimmten Implantatgröße und -höhe wird das eigentliche Zwischenwirbelimplantat ausgewählt und anschließend implantiert.

Ein Instrument der eingangs beschriebenen Art ist beispielsweise aus der WO 2004/039291 A1 bekannt.

Es ist Aufgabe der vorliegenden Erfindung, ein Instrument der eingangs beschriebenen Art so zu verbessern, daß eine Größen- und/oder Höhenbestimmung eines Zwischenwirbelimplantats auf einfache und unterschiedliche Art und Weise möglich ist.

Diese Aufgabe wird bei einem chirurgischen Instrument der eingangs beschriebenen Art erfindungsgemäß dadurch gelöst die Klemmvorrichtung ein mindestens eines der Verbindungselemente mindestens teilweise umgreifendes Klemmelement umfaßt, welches in einen Spalt zwischen dem mindestens einen Anlageelementhalteelement und dem ersten und/oder dem zweiten Probeimplantatanlageelement einsetzbar und vor dem Verbinden des mindestens einen Anlageelementhalteelements und des ersten und/oder des zweiten Probeimplantatanlageelements mindestens einseitig über den Spalt hinausragt,.

Die erfindungsgemäße Ausgestaltung ermöglicht es, die Probeimplantatanlageelemente in mindestens zwei unterschiedlichen Winkelstellungen mit dem Anlageelementhalteelement zu verbinden. Dies gestattet es, das Probeimplantat unter unterschiedlichen Winkeln in den Zwischenwirbelraum einzuführen. Mit anderen Worten ist es möglich, das Probeimplantat nicht nur durch beispielsweise einen anterioren Zugang einzuführen, sondern auch durch einen lateralen Zugang. Es kann also ein Zugang zum Zwischenwirbelraum frei gewählt werden und die Probeimplantatanlageelemente werden hierzu mit dem Halteteil entweder in einer ersten oder in einer anderen Winkelstellung mit dem Anlageelementhalteelement verbunden. Es sind somit keine Kompromisse mehr hinsichtlich der natürlichen oder optimalen Winkellage beim Einführen des Probeimplantats erforderlich. Ein Patient kann in optimaler Weise gelagert werden. Das erfindungsgemäße Instrument läßt sich dann entsprechend den operativen Gegebenheiten entsprechen einstellen. Ferner gestattet es die lösbare Verbindung der Probeimplantatanlageelemente mit dem mindestens einen Anlageelementhalteelement, unterschiedliche Probeimplantate mit unterschiedlichen Höhen und/oder Lordosewinkeln, das heißt zwischen den Anlageflächen eingeschlossenen Winkeln, mit dem Halteteil zu verbinden. Es ist also nur ein Halteteil erforderlich mit entsprechenden Probeimplantatanlageelementen, um eine Vielzahl von Probeimplantaten nachzubilden und diese durch unterschiedliche Zugänge in den Zwischenwirbelraum einzuführen. Durch die Klemmvorrichtung kann ein unbeabsichtigtes Lösen des Probeimplantatanlageelements von dem mindestens einen Anlageelementhalteelement verhindert werden. Vorteilhaft ist es, daß die Klemmvorrichtung ein mindestens eines der Verbindungselemente mindestens teilweise umgreifendes Klemmelement umfaßt, welches in einen Spalt zwischen dem mindestens einen Anlageelementhalteelement und dem ersten und/oder dem zweiten Probeimplantatanlageelement einsetzbar und vor dem Verbinden des mindestens einen Anlageelementhalteelements und des ersten und/oder des zweiten Probeimplantatanlageelements mindestens einseitig über den Spalt hinausragt. Vorzugsweise kann ein verformbares, insbesondere ein elastisches Klemmelement verwendet werden, so daß Klemmkräfte vom Klemmelement in der Verbindungsstellung in entgegengesetzter Richtung auf das Anlageelementhalteelement und das Probeimplantatanlageelement wirken.

Vorteilhaft ist es, wenn das erste oder das zweite Probeimplantatanlageelement in den mindestens zwei definierten, unterschiedlichen Winkelstellungen jeweils in einer gemeinsamen Ebene liegend angeordnet ist und daß die beiden Ebenen identisch sind. Mit anderen Worten bedeutet dies, daß das erste oder das zweite Probeimplantatanlageelement quasi durch eine rein geometrische Drehung von der ersten Winkelstellung in die zweite Winkelstellung überführbar ist. Allerdings muß das Instrument nicht so ausgebildet sein, daß der Übergang von der ersten Winkelstellung in die zweite Winkelstellung direkt durch eine Rotation möglich ist. Es kann auch eine Translations-Rotations-Translationsbewegung erforderlich sein.

Günstig ist es, wenn das erste und/oder das zweite Probeimplantatanlageelement und das mindestens eine Anlageelementhalteelement zum lösbaren Verbinden miteinander in Eingriff bringbar sind durch eine Relativbewegung aufeinander zu in einer quer zur Längsrichtung verlaufenden Verbindungsrichtung. Durch diese Ausgestaltung wird vermieden, daß beim Einführen des Probeimplantats in den Zwischenwirbelraum typischerweise in Längsrichtung wirkende Schub- und Zugkräfte dazu führen können, daß sich das Probeimplantat vom Halteteil löst.

Vorteilhafterweise ist das mindestens eine Anlageelementhalteelement mit dem ersten und/oder mit dem zweiten Probeimplantatanlageelement kraft- und/oder formschlüssig verbindbar. Es läßt sich so auf einfache Weise eine sichere Verbindung zwischen dem Anlageelementhalteelement und dem mindestens einen Probeimplantatanlageelement ausbilden.

Der Aufbau des Instrument wird besonders einfach, wenn ein erstes und ein zweites Verbindungselement vorgesehen sind zum Verbinden des mindestens einen Anlageelementhalteelements mit dem ersten und/oder mit dem zweiten Probeimplantatanlageelement und wenn eines der Probeimplantatanlageelemente und das mindestens eine Anlageelementhalteelement jeweils eines der beiden Verbindungselemente tragen. Beispielsweise durch Zusammenführen oder Ineingriffbringen der beiden Verbindungselemente kann eine sichere Verbindung zwischen dem mindestens einen Anlageelementhalteelement und dem ersten und/oder dem zweiten Probeimplantatanlageelement hergestellt werden.

Der Aufbau des Instruments wird besonders einfach, wenn das erste Verbindungselement eine Aufnahme und das zweite Verbindungselement ein zur Aufnahme korrespondierender und in diese einführbarer Vorsprung ist.

Günstig ist es, wenn der Vorsprung ein am Probeimplantatanlageelement in Richtung auf das Anlageelementhalteelement abstehender zylindrischer Zapfen ist und wenn die Aufnahme eine zum Zapfen im Durchmesser korrespondierende Bohrung oder Sacklochbohrung ist. Dies vereinfacht die Konstruktion des Instruments, seine Herstellung und auch das Austauschen der Probeimplantatanlageelemente.

Um ein unbeabsichtigtes Lösen der Probeimplantatanlageelemente vom mindestens einen Anlageelementhalteelement infolge von Schub- und Zugkräften in Längsrichtung des Halteteils zu vermeiden, ist es günstig, wenn der Vorsprung in einer Richtung quer zur Längsrichtung in die Aufnahme einführbar ist.

Günstigerweise ist eine Rastverbindung vorgesehen zum Sichern einer Verbindung des mindestens einen Anlageelementhalteelements mit dem ersten und/oder dem zweiten Probeimplantatanlageelement in einer der Winkelstellungen. Durch die Rastverbindung kann ein unbeabsichtigtes Lösen des mit dem mindestens einen Anlageelementhalteelement verbundenen Probeimplantatanlageelements in einer Verbindungsstellung verhindert werden.

Ein besonders einfacher Aufbau des Instruments ergibt sich, wenn der Spalt eine am Vorsprung in Umfangsrichtung umlaufende Ringnut umfaßt und wenn das Klemmelement ein elastischer Ring ist, dessen Durchmesser größer als eine Tiefe der Ringnut ist. Wird beispielsweise ein Verbindungszapfen mit der Ringnut versehen, so kann direkt beim Einführen des Zapfens in eine korrespondierende Aufnahme nicht nur eine Verbindung, sondern direkt auch eine Klemmung zwischen den beiden Teilen erreicht werden.

Günstigerweise ist eine Spannvorrichtung vorgesehen zum spannenden Sichern einer Verbindung des mindestens einen Anlageelementhalteelements mit dem ersten und/oder dem zweiten Probeimplantatanlageelement in einer der Winkelstellungen.

Vorteilhaft ist es, wenn eine Mehrzahl von Probeimplantaten vorgesehen ist und wenn die Mehrzahl von Probeimplantaten unterschiedliche Abstände der Anlageflächen voneinander und/oder unterschiedliche Lordosewinkel zwischen den Anlageflächen aufweisen. Durch die Mehrzahl von Probeimplantaten können mit einem einzigen Halteteil unterschiedliche Implantatgrößen und -höhen bestimmt sowie ein jeweils erforderlicher Lordosewinkel zwischen den Anlageflächen ermittelt werden. Sobald die zu bestimmenden Größen bekannt sind, kann das dauerhaft zu implantierende Zwischenwirbelimplantat ausgewählt und in den Zwischenwirbelraum eingesetzt werden.

Gemäß einer bevorzugten Ausführungsform der Erfindung kann vorgesehen sein, daß eine Winkeleinstellvorrichtung vorgesehen ist zum Vorgeben und Einstellen der unterschiedlichen Winkelstellungen des ersten und/oder des zweiten Probeimplantatanlageelements relativ zu dem mindestens einen Anlageelementhalteelement, daß die Winkeleinstellvorrichtung mindestens zwei miteinander in den unterschiedlichen Winkeleinstellungen in Eingriff bringbare Einstellelemente umfaßt und daß das erste und/oder das zweite Probeimplantatanlageelement ein erstes Einstellelement und das mindestens eine Anlageelementhalteelement ein zweites Einstellelement aufweisen. Durch die entsprechend angeordneten Einstellelemente lassen sich definierte Winkeleinstellungen des Probeimplantatanlageelements und des Anlageelementhalteelements in gewünschter Weise vorgeben.

Besonders günstig ist es, wenn mindestens eines der mindestens zwei Einstellelemente in Form einer Einstellaufnahme und wenn mindestens ein weiteres der mindestens zwei Einstellelemente in Form eines in die Einstellaufnahme einführbaren Einstellvorsprungs ausgebildet ist. Eine Winkeleinstellung kann dann auf einfache Weise vorgegeben werden, indem der Einstellvorsprung in die Einstellaufnahme eingeführt wird. Dadurch ergibt sich eine definierte Winkelstellung des Anlageelementhalteelements relativ zum Probeimplantatanlageelement.

Um eine gewünschte Winkelstellung des Probeimplantatanlageelements relativ zu dem mindestens einen Anlageelementhalteelement zu sichern, ist es vorteilhaft, wenn der Einstellvorsprung in einer Richtung quer zur Längsrichtung in die Einstellaufnahme einführbar ist. Wird das Halteteil in Längsrichtung bewegt, so können von diesem ausgeübte Schub- und Zugkräfte nicht dazu führen, daß der Einstellvorsprung relativ zur Einstellaufnahme bewegt werden kann, insbesondere aus dieser unbeabsichtigt austreten.

Der Aufbau des Instruments wird weiter vereinfacht, wenn das Probeimplantatanlageelement mindestens einen Einstellvorsprung trägt und wenn das Anlageelementhalteelement mindestens zwei Einstellaufnahmen aufweist. Dies gestattet es, den mindestens einen Einstellvorsprung des Probeimplantatanlageelements in die eine oder die andere der mindestens zwei Einstellaufnahmen einzuführen und so unterschiedliche Winkelstellungen des Probeimplantatanlageelements relativ zum mindestens einen Anlageelementhalteelement vorzugeben.

Günstig ist es, wenn das mindestens eine Anlageelementhalteelement zylindrisch geformt ist und wenn eine Längsachse des mindestens einen Anlageelementhalteelements quer zur Längsrichtung verläuft. Das Anlageelementhalteelement läßt sich so besonders einfach herstellen und auf einfache Weise mit einer Vielzahl von Probeimplantatanlageelementen verbinden.

Ferner kann es vorteilhaft sein, wenn die Probeimplantatanlageelemente jeweils eine Ausnehmung für das mindestens eine Anlageelementhalteelement aufweisen und wenn das mindestens eine Anlageelementhalteelement formschlüssig oder im wesentlichen formschlüssig in die Ausnehmung einführbar ist. Dadurch kann ein zusätzlicher Halt beziehungsweise eine Abstützung des Probeimplantatanlageelements relativ zum Anlageelementhalteelement erreicht werden.

Um das Probeimplantat durch unterschiedliche, gewünschte Zugänge im menschlichen Körper in den Zwischenwirbelraum einzuführen, ist es günstig, wenn das erste und/oder das zweite Probeimplantatanlageelement in der zweiten Winkelstellung relativ zu dem mindestens einen Anlageelementhalteelement relativ zur ersten Winkelstellung um einen Einstellwinkel verdreht anordenbar ist. Der Einstellwinkel kann jeweils so gewählt werden, daß das am Halteteil gehaltene Probeimplantat in gewünschter Weise eingeführt werden kann, zum Beispiel durch einen lateralen oder anterioren Zugang.

Günstigerweise weist der Einstellwinkel einen Wert in einem Bereich von 10° bis 30° auf.

Gemäß einer bevorzugten Ausführungsform der Erfindung kann vorgesehen sein, daß am distalen Ende des Instruments ein erstes und ein zweites Anlageelementhalteelement vorgesehen ist und daß das erste Anlageelementhalteelement mit dem ersten Probeimplantatanlageelement und das zweite Anlageelementhalteelement mit dem zweiten Probeimplantatanlageelement lösbar verbindbar ist. Dies gestattet es, die beiden Anlageelementhalteelemente individuell zum Verbinden mit den Probeimplantatanlageelementen auszubilden. Insbesondere können sie unterschiedlich geformt sein. Des weiteren ist es möglich, die Anlageelementhalteelemente relativ zueinander geneigt am Halteteil auszubilden, so daß ein Lordosewinkel bereits durch die Anlageelementhalteelemente selbst vorgebbar ist.

Um mit dem Instrument gleichzeitig auch benachbarte Wirbelkörper distrahieren zu können, ist es vorteilhaft, wenn das erste Anlageelementhalteelement und das zweite Anlageelementhalteelement quer oder im wesentlichen quer zu den Anlageflächen aufeinander zu und/oder voneinander weg bewegbar gelagert sind. Damit lassen sich einerseits die Größe des einzusetzenden Zwischenwirbelimplantats und der Lordosewinkel bestimmen sowie auch ein Abstand zwischen den Anlageflächen variieren.

Vorteilhaft kann es sein, wenn das distale Ende des Halteteils zwei Spreizbacken aufweist, wenn die Spreizbacken die Anlageelementhalteelemente umfassen oder tragen und wenn die Spreizbacken durch eine am Halteteil angeordnete Antriebsvorrichtung, welche ein am proximalen Ende des Halteteils angeordnetes Betätigungselement umfaßt, voneinander weg und/oder aufeinander zu bewegbar sind. Das Halteteil kann gemäß dieser Ausgestaltung in Form eines Spreizinstruments ausgebildet sein, welches Spreizbacken aufweist, die zum Verbinden mit den Probeimplantatanlageelementen ausgebildet sind.

Vorzugsweise sind die Spreizbacken parallel zueinander spreizbar gelagert.

Um bei einer Variation des Abstands der beiden Probeimplantatanlageelemente diesen auch bestimmen zu können, ist es vorteilhaft, wenn das Halteteil eine Wegmeßeinrichtung aufweist zum Messen und Anzeigen eines Abstandes der Anlageflächen voneinander oder eines Verfahrweges der Anlageelementhalteelemente relativ zueinander. Ist beispielsweise der Abstand der Anlageflächen voneinander bekannt, so kann mit der Wegmeßeinrichtung eine Veränderung des Abstandes bestimmt und zu dem bekannten Abstand hinzuaddiert werden. Auf diese Weise läßt sich die Zahl der erforderlichen Probeimplantatanlageelemente verringern, da unterschiedliche Höhen durch Spreizen der Anlageelementhalteelemente eingestellt und bestimmt werden können.

Die nachfolgende Beschreibung einer bevorzugten Ausführungsform der Erfindung dient im Zusammenhang mit der Zeichnung der näheren Erläuterung. Es zeigen:
- Figur 1:: ein distales Ende eines erfindungsgemäßen Instruments mit zwischen benachbarten Wirbelkörpern eingesetzten Probeimplantatplatten;
- Figur 2:: eine vergrößerte Darstellung des distalen Endes des Instruments mit in einer ersten Winkelstellung am distalen Ende des Instruments gehaltenen Probeimplantatplatten;
- Figur 3:: eine Ansicht ähnlich Figur 2, jedoch mit den in einer zweiten Winkeleinstellung am distalen Ende des Instruments gehaltenen Probeimplantatplatten; und
- Figur 4:: eine Explosionsdarstellung des distalen Endes des Instruments.

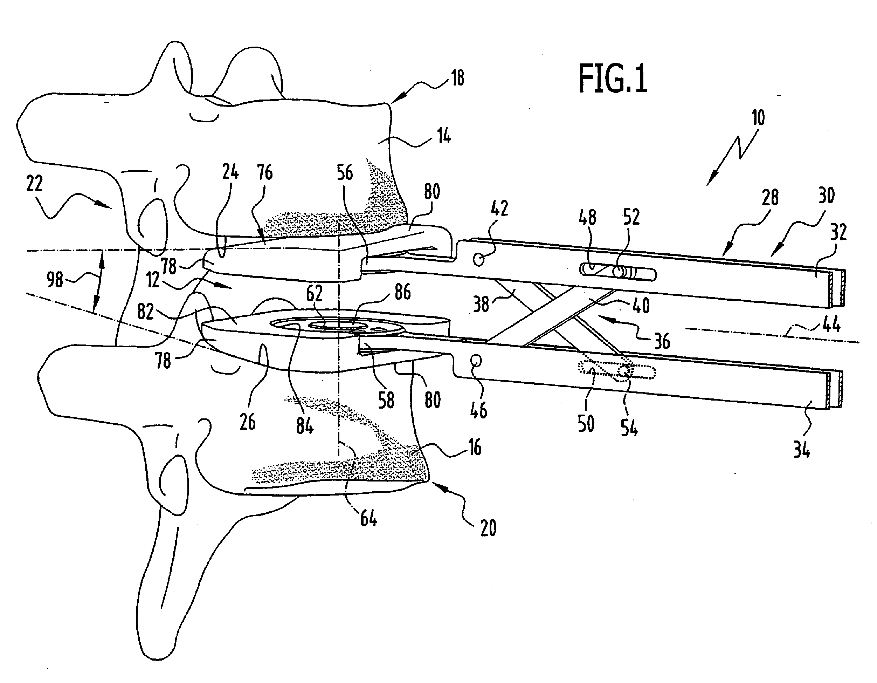

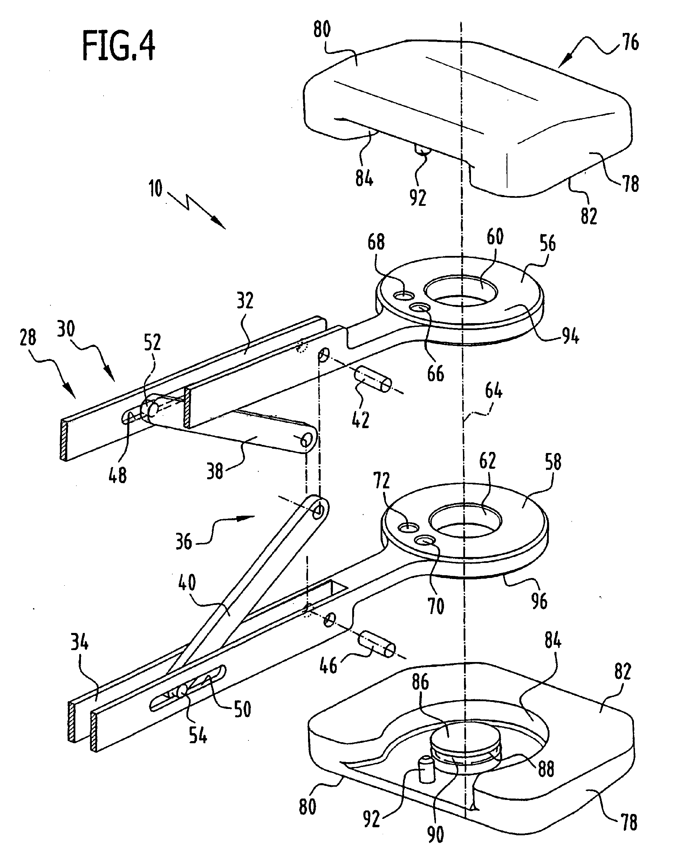

In den Figuren 1 bis 4 ist ein insgesamt mit dem Bezugszeichen 10 versehenes chirurgisches Instrument zur Größen- und/oder Höhenbestimmung eines in einen Zwischenwirbelraum 12 zwischen zwei benachbarte Wirbelkörper 14 und 16 zweier Wirbel 18 und 20 einer Wirbelsäule 22 einsetzbaren Zwischenwirbelimplantats. Das Zwischenwirbelimplantat ist in den Figuren nicht dargestellt, umfaßt jedoch typischerweise ein erstes Anlageelement zur Anlage an eine erste Gelenkfläche 24 des Wirbelkörpers 14 und ein am ersten Anlageelement direkt oder indirekt gelagertes, insbesondere beweglich gelagertes, zweites Anlageelement zur Anlage an eine zweite, in Richtung auf die erste Gelenkfläche 24 hin weisende zweite Gelenkfläche 26 des Wirbelkörpers 16.

Das Instrument 10 umfaßt einen als Halteteil ausgebildeten Instrumententeil 28, an dessen distalem Ende eine Spreizvorrichtung 30 angeordnet ist. Die Spreizvorrichtung 30 umfaßt zwei parallel zueinander ausgerichtete Spreizbacken 32 und 34 die über ein Scherenglied 36 miteinander verbunden sind. Das Scherenglied 36 umfaßt zwei Lenker 38 und 40, wobei der Lenker 38 mit einem Lagerstift 42, dessen Längsachse quer zu einer Längsachse 44 des Instrumententeils 28 orientiert ist, mit der Spreizbacke 32 schwenkbar verbunden ist. In gleicher Weise ist der Lenker 40 an einem distalen Ende der Spreizbacke 34 mittels eines parallel zum Lagerstift 42 orientierten Lagerstifts 46 schwenkbar gelagert. Jede der beiden Spreizbacken 32 und 34 ist mit einem parallel zur Längsachse 44 verlaufenden Langloch 48 beziehungsweise 50 versehen, in welches ein an den freien Enden der Lenker 38 und 40 quer zur Längsachse abstehender Führungszapfen 52 beziehungsweise 54 eintauchen und geführt werden. Durch das Scherenglied 36 wird eine parallele Bewegung der beiden Spreizbacken 32 und 34 erzwungen. Die Spreizvorrichtung 30 bildet so einen Parallelspreizer.

Distalseitig ist an den Spreizbacken 32 und 34 jeweils ein Haltering 56 und 58 angeordnet, die Anlageelementhalteelemente bilden und jeweils eine zentrale Bohrung 60 beziehungsweise 62 aufweisen, die als Aufnahmen dienen. Eine Symmetrieachse 64 der Halteringe 56 und 58 verläuft quer zur Längsachse 44. Ferner sind die Halteringe 56 und 58 jeweils zweimal durchbohrt, und zwar parallel zur Symmetrieachse 64. Es sind somit jeweils zwei Justierbohrungen 66 und 68 sowie 70 und 72 in jedem Haltering 56 und 58 ausgebildet. Längsachsen der Bohrung 60 und der Justierbohrung 66 schneiden beide die Längsachse 44, ebenso Längsachsen der Bohrung 62 und der Justierbohrung 70. Die Justierbohrungen 68 und 72 sind relativ zu den Justierbohrungen 66 und 70 um einen Einstellwinkel 74 in Umfangsrichtung versetzt angeordnet, der beim vorliegenden Ausführungsbeispiel 22° beträgt.

Teil des Instruments 10 ist auch ein Probeimplantat 76, welches zwei identische Platten 78 umfaßt. Die Platte 78 ist im wesentlichen quaderförmig ausgebildet und weist eine zur Anlage an eine der Gelenkflächen 24 beziehungsweise 26 anlegbare Anlagefläche 80 auf. Auf eine im wesentlichen parallel zur Anlagefläche 80 in entgegengesetzter Richtung weisenden Unterseite 82 ist eine zylindrische Ringaufnahme 84 eingearbeitet, welche im wesentlichen in proximaler Richtung weisend seitlich geöffnet ist. Der Durchmesser der Ringaufnahme 84 entspricht einem Außendurchmesser der Halteringe 56 und 58. Der Ringaufnahme 84 ist koaxial zur Symmetrieachse 64 angeordnet. Ebenfalls koaxial zu derselben steht ein Haltezapfen 86 von der Platte 78 ab, dessen Außendurchmesser dem Innendurchmesser der Bohrungen 60 und 62 entspricht. Der Haltezapfen 86 ist in Umfangsrichtung mit einer Ringnut 88 versehen, in welche ein O-Ring 90 eingelegt ist, dessen Durchmesser etwas größer ist als eine Tiefe der Ringnut 88 in radialer Richtung. Die Ringnut 88 mit dem in diese eingelegten O-Ring 90 bilden eine Klemmvorrichtung, mit welcher eine Verbindung der Platte 78 mit einem der beiden Halteringe 56 und 58 gesichert werden kann. Eine Klemmung erfolgt durch elastische Verformung des O-Rings 90 nach Einführen des Haltezapfens 86 in eine der beiden Bohrungen 60 oder 62. Parallel zum Haltezapfen 86 steht ein Einstellzapfen 92 ab, dessen Außendurchmesser den Innendurchmessern der Justierbohrungen 66 bis 72 entspricht.

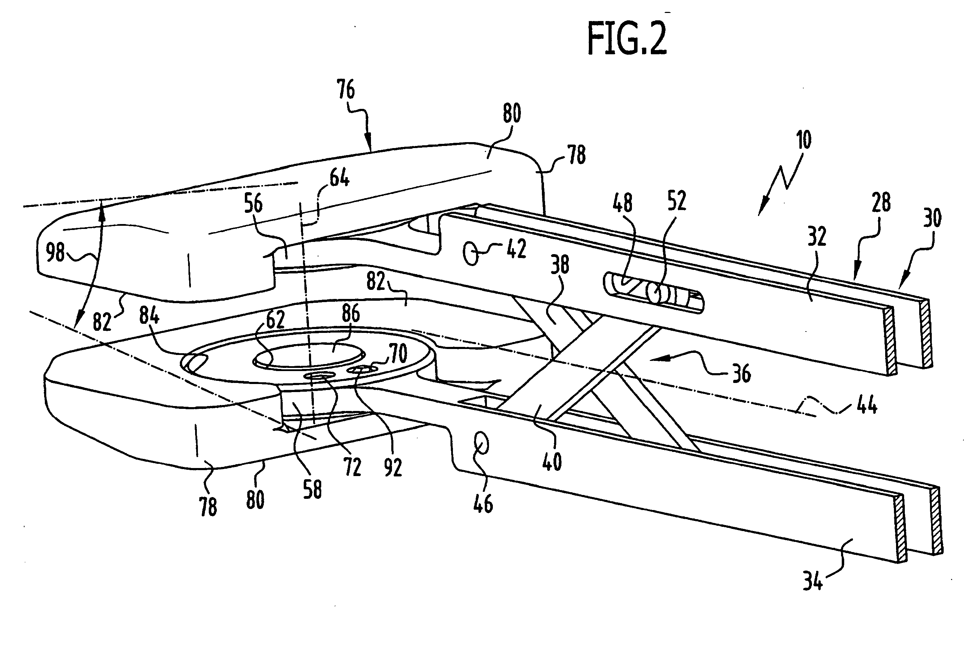

Die Platte 78 ist derart ausgebildet, daß sie einen der Halteringe 56 oder 58 aufnehmen kann, wobei der Haltezapfen 86 in die Bohrung 60 beziehungsweise 62 und der Einstellzapfen 92 in eine der Justierbohrungen 66 und 68 beziehungsweise 70 und 72 einführbar ist. Die Platte 78 kann somit auf einfache Weise parallel zur Symmetrieachse 64, also quer zur Längsachse 44, mit einem der Halteringe 56 und 58 in Eigriff gebracht werden. Sind die beiden Platten 78 mit den Halteringen 56 und 58 derart verbunden, daß die Einstellzapfen 92 in die Justierbohrungen 66 und 70 eintauchen, dann ergibt sich eine Ausrichtung der Platten 78 symmetrisch zum Instrumententeil 28 beziehungsweise zur Längsachse 44. Mit einer derartigen, in den Figuren 1 und 2 dargestellten Winkeleinstellung der Platten 78 relativ zu den Halteringen 56 und 58 läßt sich das Instrument dazu verwenden, das Probeimplantat 76 durch einen anterioren Zugang in einen menschlichen oder tierischen Körper und in den Zwischenwirbelraum 12 einzuführen.

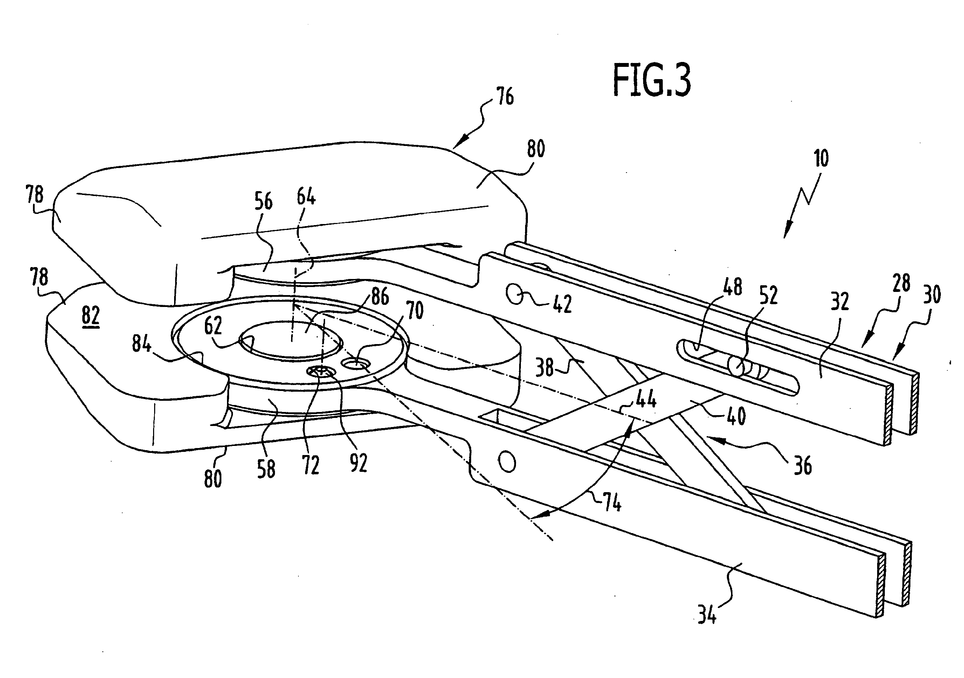

Das Einführen des Probeimplantats 76 durch einen lateralen Zugang wird erleichtert, wenn die Platten 78 mit den Halteringen 56 und 58 derart verbunden werden, daß die Einstellzapfen 92 in die Justierbohrungen 68 und 72 eintauchen. Die Platten 78 sind so einer in Figur 3 dargestellten zweiten Winkelstellung mit den Halteringen 56 und 58 verbunden, und nehmen ein im Vergleich zu der in den Figuren 1 und 2 dargestellten Winkelstellung eine um 22° gedrehte Stellung ein.

Bei dem in den Figuren 1 bis 4 dargestellten Ausführungsbeispiel sind die breiten Halteringe 56 und 58 parallel zueinander ausgerichtet. Denkbar wäre es auch, voneinander weg weisende Ringflächen 94 und 96 der Halteringe 56 und 58 gegeneinander zu neigen. Auf diese Weise läßt sich ein bestimmter Lordosewinkel 98, den die Gelenkflächen 24 und 26 relativ zueinander vorgeben, einstellen.

Das Instrument 10 umfaßt nicht nur die in den Figuren 1 bis 4 dargestellten Platten 78, sondern auch weitere, nicht dargestellte Platten 78 unterschiedlicher Größe und unterschiedlicher Neigung der Anlageflächen 80 relativ zueinander. Durch die unterschiedlich geneigten Anlageflächen 80 kann ebenfalls der durch die Gelenkflächen 24 und 26 vorgegebene Lordosewinkel 98 nachgebildet werden. Des weiteren unterscheiden sich die Platten 78 auch in ihrer Höhe, so daß unterschiedlich hohe Zwischenwirbelräume 12 in gewünschter Weise ausgemessen werden können.

Das Instrument 10 wird intraoperativ wie folgt eingesetzt. Zunächst wird ein Patient in gewünschter Weise gelagert. Dies kann beispielsweise bei einem frontalen Zugang auf dem Rücken liegend erfolgen. Über die spezielle Lagerung des Patients kann bereits eine Entlastung der Wirbelsäule erreicht werden.

In einem nächsten Schritt wird ein Zugang in den Körper des Patienten geschaffen, und zwar zum gewünschten Zwischenwirbelraum 12 hin. Anschließend wird das geschädigte Bandscheibengewebe entfernt. Danach wird das Instrument 10 entsprechend vorbereitet. Dies bedeutet, daß anhand der anatomischen Verhältnisse verschiedene Probeimplantate 76 etwa passender Größe vorbereitet werden, und zwar mit entsprechender Höhe und natürlichem Lordosewinkel 98. Entsprechend dem gewählten Zugang, anterior oder lateral, werden die Platten 78 des Probeimplantats 76 auf die jeweiligen Halteringe 56 und 58 aufgesteckt, also entweder mit den Einstellzapfen 92 in die Justierbohrungen 66 und 70 oder 68 und 72. Danach wird das Instrument 10 in den Körper des Patienten eingeführt, und zwar derart, daß das Probeimplantat 76 in den Zwischenwirbelraum 12 eintaucht. Mit einer nicht näher dargestellten Antriebsvorrichtung werden, falls erforderlich, die Spreizbacken 32 und 34 auseinanderbewegt, bis die Anlageflächen 80 an den Gelenkflächen 24 und 26 anliegen. Bei Bedarf können mit dem Instrument 10 die benachbarten Wirbelkörper 14 und 16 noch weiter aufgespreizt werden. Am Instrumententeil 28 ist eine nicht dargestellte Anzeigevorrichtung ausgebildet, welche eine Skala umfaßt, an der bestimmte Implantatgrößen gekennzeichnet sind. Bei entsprechender Auswahl der Platten 78 kann dann direkt an der Skala das zu wählende Implantat abgelesen werden. Alternativ kann die Skala auch eine Längenskala sein, so daß anhand des angezeigten Werts ein Abstand der beiden Gelenkflächen 24 und 26 voneinander bestimmt und dementsprechend das einzusetzende Zwischenwirbelimplantat gewählt werden kann.

In einem nächsten Schritt werden die beiden Spreizbacken 32 und 34 wieder aufeinander zu bewegt und das Probeimplantat 76 aus dem Zwischenwirbelraum 12 entfernt.

## Patentansprüche

1. Chirurgisches Instrument (10) zur Größen- und/oder Höhenbestimmung eines in einen Zwischenwirbelraum (12) zwischen zwei benachbarte Wirbelkörper (14, 16) zweier Wirbel (18, 20) einer menschlichen oder tierischen Wirbelsäule (22) einsetzbaren Zwischenwirbelimplantats, wobei das Zwischenwirbelimplantat ein erstes Anlageelement zur Anlage an eine Gelenkfläche (24) des einen (14) der zwei benachbarten Wirbelkörper (14, 16) und ein am ersten Anlageelement direkt oder indirekt gelagertes zweites Anlageelement zur Anlage an eine Gelenkfläche (26) des anderen (16) der zwei benachbarten Wirbelkörper (14, 16) aufweist, wobei das Instrument (10) ein eine Längsrichtung (44) definierendes Halteteil (28) und mindestens ein Probeimplantat (76) umfaßt, welches ein erstes und ein zweites, jeweils mindestens eine Anlagefläche (80) zur Anlage an einer der Gelenkflächen (24, 26) aufweisendes Probeimplantatanlageelement (78) umfaßt, wobei an einem distalen Ende des Halteteils (28) mindestens ein Anlageelementhalteelement (56, 58) zum lösbaren Verbinden mit dem mindestens einen Probeimplantat (76) vorgesehen ist, wobei das erste und/oder das zweite Probeimplantatanlageelement (78) bezogen auf die Längsrichtung (44) in mindestens zwei definierten, unterschiedlichen Winkelstellungen relativ zu dem mindestens einen Anlageelementhalteelement (56, 58) mit diesem lösbar verbindbar ist und wobei eine Klemmvorrichtung (88, 90) vorgesehen ist zum klemmenden Sichern einer Verbindung des mindestens einen Anlageelementhalteelements (56, 58) mit dem ersten und/oder dem zweiten Probeimplantatanlageelement (78) in einer der Winkelstellungen, **dadurch gekennzeichnet, daß** die Klemmvorrichtung ein mindestens eines der Verbindungselemente (60, 62, 86) mindestens teilweise umgreifendes Klemmelement (90) umfaßt, welches in einen Spalt (88) zwischen dem mindestens einen Anlageelementhalteelement (56, 58) und dem ersten und/ oder dem zweiten Probeimplantatanlageelement (78) einsetzbar ist und vor dem Verbinden des mindestens einen Anlageelementhalteelements (56, 58) und des ersten und/oder des zweiten Probeimplantatanlageelements (78) mindestens einseitig über den Spalt (88) hinausragt.

2. Instrument nach Anspruch 1, **dadurch gekennzeichnet, daß** das erste oder das zweite Probeimplantatanlageelement (78) in den mindestens zwei definierten, unterschiedlichen Winkelstellungen jeweils in einer Ebene liegend angeordnet ist und daß die beiden Ebenen identisch sind.

3. Instrument nach einem der voranstehenden Ansprüche, **dadurch gekennzeichnet, daß** das erste und/oder das zweite Probeimplantatanlageelement (78) und das mindestens eine Anlageelementhalteelement (56, 58) zum lösbaren Verbinden miteinander in Eingriff bringbar sind durch eine Relativbewegung aufeinander zu in einer quer zur Längsrichtung (44) verlaufenden Verbindungsrichtung (64).

4. Instrument nach einem der voranstehenden Ansprüche, **dadurch gekennzeichnet, daß** das mindestens eine Anlageelementhalteelement (56, 58) mit dem ersten und/oder dem zweiten Probeimplantatanlageelement (78) kraft- und/oder formschlüssig verbindbar ist.

5. Instrument nach einem der voranstehenden Ansprüche, **dadurch gekennzeichnet, daß** ein erstes und ein zweites Verbindungselement (60, 62, 86) vorgesehen sind zum Verbinden des mindestens einen Anlageelementhalteelements (56, 58) mit dem ersten und/oder dem zweiten Probeimplantatanlageelement (78) und daß eines der Probeimplantatanlageelemente (78) und das mindestens eine Anlageelementhalteelement (56, 58) jeweils eines der beiden Verbindungselemente (60, 62, 86) tragen.

6. Instrument nach Anspruch 5, **dadurch gekennzeichnet, daß** das erste Verbindungselement eine Aufnahme (60, 62) und das zweite Verbindungselement ein zur Aufnahme (60, 62) korrespondierender und in diese einführbarer Vorsprung (86) ist.

7. Instrument nach Anspruch 6, **dadurch gekennzeichnet, daß** der Vorsprung ein am Probeimplantatanlageelement in Richtung auf das Anlageelementhalteelement (56, 58) abstehender zylindrischer Zapfen (86) ist und daß die Aufnahme eine zum Zapfen (86) im Durchmesser korrespondierende Bohrung (60, 62) oder Sachlochbohrung ist.

8. Instrument nach einem der Ansprüche 5 oder 6, **dadurch gekennzeichnet, daß** der Vorsprung (86) in einer Richtung (64) quer zur Längsrichtung (44) in die Aufnahme (60, 62) einführbar ist.

9. Instrument nach einem der voranstehenden Ansprüche, **dadurch gekennzeichnet, daß** eine Rastverbindung vorgesehen ist zum Sichern einer Verbindung des mindestens einen Anlageelementhalteelements (56, 58) mit dem ersten und/oder dem zweiten Probeimplantatanlageelement (78) in einer der Winkelstellungen.

10. Instrument nach einem der voranstehenden Ansprüche, **dadurch gekennzeichnet, daß** der Spalt eine am Vorsprung (86) in Umfangsrichtung umlaufende Ringnut (88) umfaßt und daß das Klemmelement ein elastischer Ring (90) ist, dessen Durchmesser größer als eine Tiefe der Ringnut (88) ist.

11. Instrument nach einem der voranstehenden Ansprüche, **dadurch gekennzeichnet, daß** eine Spannvorrichtung vorgesehen ist zum spannenden Sichern einer Verbindung des mindestens einen Anlageelementhalteelements (56, 58) mit dem ersten und/oder dem zweiten Probeimplantatanlageelement (78) in einer der Winkelstellungen.

12. Instrument nach einem der voranstehenden Ansprüche, **dadurch gekennzeichnet, daß** eine Mehrzahl von Probeimplantaten (76) vorgesehen ist und daß die Mehrzahl von Probeimplantaten (78) unterschiedliche Abstände der Anlageflächen (80) voneinander und/oder unterschiedliche Lordosewinkel (98) zwischen den Anlageflächen (80) aufweisen.

13. Instrument nach einem der voranstehenden Ansprüche, **dadurch gekennzeichnet, daß** eine Winkeleinstellvorrichtung (66, 68, 70, 72, 92) vorgesehen ist zum Vorgeben und Einstellen der unterschiedlichen Winkelstellungen des ersten und/oder des zweiten Probeimplantatanlageelements (78) relativ zu dem mindestens einen Anlageelementhalteelement (56, 58), daß die Winkeleinstellvorrichtung (66, 68, 70, 72, 92) mindestens zwei miteinander in den unterschiedlichen Winkelstellungen in Eingriff bringbare Einstellelemente (66, 68, 70, 72, 92) umfaßt und daß das erste und/oder das zweite Probeimplantatanlageelement (78) ein erstes Einstellelement (92) und das mindestens eine Anlageelementhalteelement (56, 58) ein zweites Einstellelement (66, 68, 70, 72) aufweisen.

14. Instrument nach Anspruch 13, **dadurch gekennzeichnet, daß** mindestens eines der mindestens zwei Einstellelemente (66, 68, 70, 72, 92) in Form einer Ei.nstellaufnahme (66, 68, 70, 72) und daß mindestens ein weiteres der mindestens zwei Einstellelemente (66, 68, 70, 72, 92) in Form eines in die Einstellaufnahme (66, 68, 70, 72) einführbaren Einstellvorsprungs (92) ausgebildet ist.

15. Instrument nach Anspruch 14, **dadurch gekennzeichnet, daß** der Einstellvorsprüng (92) in einer Richtung (64) quer zur Längsrichtung (44) in die Einstellaufnahme (66, 68, 70, 72) einführbar ist.

16. Instrument nach einem der Ansprüche 14 oder 15, **dadurch gekennzeichnet, daß** das Probeimplantatanlageelement (78) mindestens einen Einstellvorsprung (92) trägt und daß das Anlageelementhalteelement (56, 58) mindestens zwei Einstellaufnahmen (66, 68, 70, 72) aufweist.

17. Instrument nach einem der voranstehenden Ansprüche, **dadurch gekennzeichnet, daß** das mindestens eine Anlageelementhalteelement (56, 58) zylindrisch geformt ist und daß eine Längsachse (64) des mindestens einen Anlageelementhalteelements (56, 58) quer zur Längsrichtung (44) verläuft.

18. Instrument nach einem der voranstehenden Ansprüche, **dadurch gekennzeichnet, daß** die Probeimplantatanlageelemente (78) jeweils eine Ausnehmung für das mindestens eine Anlageelementhalteelement (56, 58) aufweisen und daß das mindestens eine Anlageelementhalteelement (56, 58) formschlüssig oder im wesentlichen formschlüssig in die Ausnehmung (84) einführbar ist.

19. Instrument nach einem der voranstehenden Ansprüche, **dadurch gekennzeichnet, daß** das erste und/oder das zweite Probeimplantatanlageelement (78) in der zweiten Winkelstellung relativ zu dem mindestens einen Anlageelementhalteelement (56, 58) relativ zur ersten Winkelstellung um einen Einstellwinkel (74) verdreht anordenbar ist.

20. Instrument nach Anspruch 19, **dadurch gekennzeichnet, daß** der Einstellwinkel (74) einen Wert in einem Bereich von 10° bis 30° aufweist.

21. Instrument nach einem der voranstehenden Ansprüche, **dadurch gekennzeichnet, daß** am distalen Ende des Instruments (10) ein erstes und ein zweites Anlageelementhalteelement (56, 58) vorgesehen sind und daß das erste Anlageelementhalteelement (56) mit dem ersten Probeimplantatanlageelement (78) und das zweite Anlageelementhalteelement (58) mit dem zweiten Probeimplantatanlageelement (78) lösbar verbindbar sind.

22. Instrument nach Anspruch 21, **dadurch gekennzeichnet, daß** das erste Anlageelementhalteelement (56) und das zweite Anlageelementhalteelement (58) quer oder im wesentlichen quer zu ihren Anlageflächen (80) aufeinander zu und/oder voneinander weg bewegbar gelagert sind.

23. Instrument nach einem der Ansprüche 21 oder 22, **dadurch gekennzeichnet, daß** das distale Ende des Halteteils (28) zwei Spreizbacken (32, 34) aufweist, daß die Spreizbacken (32, 34) die Anlageelementhalteelemente (56, 58) umfassen oder tragen und daß die Spreizbacken (32, 34) durch eine am Halteteil (28) angeordnete Antriebsvorrichtung, welche ein am proximalen Ende des Halteteils (28) angeordnetes Betätigungselement umfaßt, voneinander weg und/oder aufeinander zu bewegbar sind.

24. Instrument nach Anspruch 23, **dadurch gekennzeichnet, daß** die Spreizbacken (32, 34) parallel zueinander spreizbar gelagert sind.

25. Instrument nach einem der Ansprüche 22 bis 24, **dadurch gekennzeichnet, daß** das Halteteil (28) eine Wegmesseinrichtung aufweist zum Messen und Anzeigen eines Abstandes der Anlageflächen (80) voneinander oder eines Verfahrweges der Anlageelementhalteelemente (56, 58) relativ zueinander.

## Claims

1. Surgical instrument (10) for determining the size and/or height of an intervertebral implant insertable into an intervertebral space (12) between two adjacent vertebral bodies (14, 16) of two vertebrae (18, 20) of a human or animal spinal column (22), wherein the intervertebral implant comprises a first abutment element for abutment against an articular surface (24) of one (14) of the two adjacent vertebral bodies (14, 16) as well as a second abutment element supported directly or indirectly on the first abutment element for abutment against an articular surface (26) of the other (16) of the two adjacent vertebral bodies (14, 16), wherein the instrument (10) comprises a holding part (28) defining a longitudinal direction (44) and at least one test implant (76), which comprises a first and a second test-implant abutment element (78) each having at least one abutment surface (80) for abutment against one of the articular surfaces (24, 26), wherein provided on a distal end of the holding part (28) is at least one abutment-element holding element (56, 58) for detachable connection to the at least one test implant (76), wherein the first and/or the second test-implant abutment element (78) in relation to the longitudinal direction (44) is detachably connectable to the at least one abutment-element holding element (56, 58) in at least two defined, different angular positions relative thereto, and wherein a clamping device (88, 90) is provided for clamping and securing a connection of the at least one abutment-element holding element (56, 58) to the first and/or the second test-implant abutment element (78) in one of the angular positions, **characterized in that** the clamping device comprises a clamping element (90), which at least partially embraces at least one of the connection elements (60, 62, 86) and which can be inserted into a gap (88) between the at least one abutment-element holding element (56, 58) and the first and/or the second test-implant abutment element (78) and before the connection of the at least one abutment-element holding element (56, 58) and the first and/or the second test-implant abutment element (78) projects at least at one side beyond the gap (88).

2. Instrument according to claim 1, **characterized in that** the first or the second test-implant abutment element (78) in the at least two defined, different angular positions is disposed lying in each case in one plane and that the two planes are identical.

3. Instrument according to one of the preceding claims, **characterized in that** the first and/or the second test-implant abutment element (78) and the at least one abutment-element holding element (56, 58), for detachable connection to one another, can be brought into engagement by means of a relative movement towards one another in a connection direction (64) extending transversely of the longitudinal direction (44).

4. Instrument according to one of the preceding claims, **characterized in that** the at least one abutment-element holding element (56, 58) is non-positively and/or positively connectable to the first and/or the second test-implant abutment element (78).

5. Instrument according to one of the preceding claims, **characterized in that** a first and a second connection element (60, 62, 86) are provided for connecting the at least one abutment-element holding element (56, 58) to the first and/or the second test-implant abutment element (78) and that one of the test-implant abutment elements (78) and the at least one abutment-element holding element (56, 58) each carry one of the two connection elements (60, 62, 86).

6. Instrument according to claim 5, **characterized in that** the first connection element is a receiver (60, 62) and the second connection element is a projection (86), which corresponds to the receiver (60, 62) and can be introduced therein.

7. Instrument according to claim 6, **characterized in that** the projection is a cylindrical pin (86), which projects from the test-implant abutment element in the direction of the abutment-element holding element (56, 58) and that the receiver is a bore (60, 62) or blind hole bore corresponding in diameter to the pin (86).

8. Instrument according to one of claims 5 or 6, **characterized in that** the projection (86) can be introduced into the receiver (60, 62) in a direction (64) transversely of the longitudinal direction (44).

9. Instrument according to one of the preceding claims, **characterized in that** a detent connection is provided for securing a connection of the at least one abutment-element holding element (56, 58) to the first and/or the second test-implant abutment element (78) in one of the angular positions.

10. Instrument according to one of the preceding claims, **characterized in that** the gap comprises an annular groove (88) running in peripheral direction around the projection (86) and that the clamping element is an elastic ring (90), the diameter of which is larger than a depth of the annular groove (88).

11. Instrument according to one of the preceding claims, **characterized in that** a tensioning device is provided for tensioning and securing a connection of the at least one abutment-element holding element (56, 58) to the first and/or the second test-implant abutment element (78) in one of the angular positions.

12. Instrument according to one of the preceding claims, **characterized in that** a plurality of test implants (76) is provided and that the plurality of test implants (78) has different spacings between the abutment surfaces (80) and/or different lordosis angles (98) between the abutment surfaces (80).

13. Instrument according to one of the preceding claims, **characterized in that** an angle setting device (66, 68, 70, 72, 92) is provided for defining and setting the different angular positions of the first and/or the second test-implant abutment element (78) relative to the at least one abutment-element holding element (56, 58), that the angle setting device (66, 68, 70, 72, 92) comprises at least two setting elements (66, 68, 70, 72, 92) that can be brought into engagement with one another in the different angular positions, and that the first and/or the second test-implant abutment element (78) has a first setting element (92) and the at least one abutment-element holding element (56, 58) has a second setting element (66, 68, 70, 72).

14. Instrument according to claim 13, **characterized in that** at least one of the at least two setting elements (66, 68, 70, 72, 92) is designed in the form of a setting receiver (66, 68, 70, 72) and that at least one other of the at least two setting elements (66, 68, 70, 72, 92) is designed in the form of a setting projection (92) which can be introduced into the setting receiver (66, 68, 70, 72).

15. Instrument according to claim 14, **characterized in that** the setting projection (92) can be introduced into the setting receiver (66, 68, 70, 72) in a direction (64) transversely of the longitudinal direction (44).

16. Instrument according to one of claims 14 or 15, **characterized in that** the test-implant abutment element (78) carries at least one setting projection (92) and that the abutment-element holding element (56, 58) has at least two setting receivers (66, 68, 70, 72).

17. Instrument according to one of the preceding claims, **characterized in that** the at least one abutment-element holding element (56, 58) is cylindrical in shape and that a longitudinal axis (64) of the at least one abutment-element holding element (56, 58) extends transversely of the longitudinal direction (44).

18. Instrument according to one of the preceding claims, **characterized in that** the test-implant abutment elements (78) each have a recess for the at least one abutment-element holding element (56, 58) and that the at least one abutment-element holding element 56, 58) can be introduced positively or substantially positively into the recess (84).

19. Instrument according to one of the preceding claims, **characterized in that** the first and/or the second test-implant abutment element (78) can be disposed in the second angular position relative to the at least one abutment-element holding element (56, 58) by being rotated through a setting angle (74) relative to the first angular position.

20. Instrument according to claim 19, **characterized in that** the setting angle (74) has a value in a range of 10° to 30°.

21. Instrument according to one of the preceding claims, **characterized in that** a first and a second abutment-element holding element (56, 58) are provided on the distal end of the instrument (10) and that the first abutment-element holding element (56) is detachably connectable to the first test-implant abutment element (78) and the second abutment-element holding element (58) is detachably connectable to the second test-implant abutment element (78).

22. Instrument according to claim 21, **characterized in that** the first abutment-element holding element (56) and the second abutment-element holding element (58) are supported such that they are movable towards and/or away from one another transversely or substantially transversely of their abutment surfaces (80).

23. Instrument according to one of claims 21 or 22, **characterized in that** the distal end of the holding part (28) has two spreading jaws (32, 34), that the spreading jaws (32, 34) comprise or carry the abutment-element holding elements (56, 58) and that the spreading jaws (32, 34) are movable away from and/or towards one another by means of a drive device, which is disposed on the holding part (28) and comprises an actuating element disposed on the proximal end of the holding part (28).

24. Instrument according to claim 23, **characterized in that** the spreading jaws (32, 34) are supported such that they can be spread parallel to one another.

25. Instrument according to one of claims 22 to 24, **characterized in that** the holding part (28) has a path measuring device for measuring and indicating a spacing between the abutment surfaces (80) or a displacement of the abutment-element holding elements (56, 58) relative to one another.

## Revendications

1. Instrument chirurgical (10) pour la détermination de la grandeur et/ou de la hauteur d'un implant intervertébral pouvant être inséré dans un espace intervertébral (12) entre deux corps de vertèbre (14, 16) voisins de deux vertèbres (18, 20) d'une colonne vertébrale (22) humaine ou animale,
l'implant intervertébral présentant un premier élément d'appui destiné à venir s'appuyer sur une surface d'articulation (24) de l'un (14) des deux corps de vertèbre (14, 16) voisins, et un deuxième élément d'appui monté directement ou indirectement sur le premier élément d'appui et destiné à venir s'appuyer sur une surface d'articulation (26) de l'autre (16) des deux corps de vertèbre (14, 16) voisins,
l'instrument (10) comprenant une pièce de maintien (28), qui définit une direction longitudinale (44), et au moins un implant probatoire (76), qui comprend un premier et un deuxième élément d'appui d'implant probatoire (78), qui présentent chacun au moins une surface d'appui (80) destinée à venir s'appuyer sur l'une des surfaces d'articulation (24, 26),
au moins un élément de maintien d'élément d'appui (56, 58) étant prévu à une extrémité distale de la pièce de maintien (28) pour la liaison amovible avec ledit au moins un implant probatoire (76),
le premier et/ou le deuxième élément d'appui d'implant probatoire (78) pouvant être relié de manière amovible au dit au moins un élément de maintien d'élément d'appui (56, 58), dans au moins deux positions angulaires définies, différentes, par rapport à celui-ci, en se référant à la direction longitudinale (44),
et un dispositif de coincement (88, 90) étant prévu pour la sécurisation par coincement d'une liaison dudit au moins un élément de maintien d'élément d'appui (56, 58) avec le premier et/ou le deuxième élément d'appui d'implant probatoire (78) dans l'une des positions angulaires,
**caractérisé en ce que** le dispositif de coincement comprend un élément de coincement (90) qui entoure au moins partiellement au moins l'un des éléments de liaison (60, 62, 86), et qui peut être inséré dans une fente (88) entre ledit au moins un élément de maintien d'élément d'appui (56, 58) et le premier et/ou le deuxième élément d'appui d'implant probatoire (78), et qui, avant l'établissement de la liaison dudit moins un élément de maintien d'élément d'appui (56, 58) et du premier et/ou du deuxième élément d'appui d'implant probatoire (78), fait saillie hors de la fente (88), au moins d'un côté.

2. Instrument selon la revendication 1, **caractérisé en ce que** le premier ou le deuxième élément d'appui d'implant probatoire (78) est agencé, dans lesdites au moins deux positions angulaires définies, différentes, de manière à être situé à chaque fois dans un plan, et **en ce que** les deux plans sont identiques.

3. Instrument selon l'une des revendications précédentes, **caractérisé en ce que** le premier et/ou le deuxième élément d'appui d'implant probatoire (78) et ledit au moins un élément de maintien d'élément d'appui (56, 58) peuvent, pour leur liaison amovible, être amenés en prise réciproque, par un mouvement relatif de l'un vers l'autre dans une direction de liaison (64) s'étendant transversalement à la direction longitudinale (44).

4. Instrument selon l'une des revendications précédentes, **caractérisé en ce que** ledit au moins un élément de maintien d'élément d'appui (56, 58) peut être relié par adhérence et/ou par complémentarité formes avec le premier et/ou le deuxième élément d'appui d'implant probatoire (78).

5. Instrument selon l'une des revendications précédentes, **caractérisé en ce que** sont prévus un premier et un deuxième élément de liaison (60, 62, 86) pour relier ledit au moins un élément de maintien d'élément d'appui (56, 58) avec le premier et/ou le deuxième élément d'appui d'implant probatoire (78), et **en ce que** l'un des éléments d'appui d'implant probatoire (78) et ledit au moins un élément de maintien d'élément d'appui (56, 58) portent respectivement un des deux éléments de liaison (60, 62, 86).

6. Instrument selon la revendication 5, **caractérisé en ce que** le premier élément de liaison est un logement de réception (60, 62), et le deuxième élément de liaison est une protubérance (86) en correspondance avec le logement de réception (60, 62) et pouvant être introduite dans celui-ci.

7. Instrument selon la revendication 6, **caractérisé en ce que** la protubérance est un tenon cylindrique (86) faisant saillie de l'élément d'appui d'implant probatoire en direction de l'élément de maintien d'élément d'appui (56, 58), et **en ce que** le logement de réception est un alésage (60, 62) ou alésage borgne d'un diamètre correspondant à celui du tenon (86).

8. Instrument selon l'une des revendications 5 ou 6, **caractérisé en ce que** la protubérance (86) peut être introduite dans le logement de réception (60, 62), dans une direction (64) transversale à la direction longitudinale (44).

9. Instrument selon l'une des revendications précédentes, **caractérisé en ce qu'**il est prévu une liaison par encliquetage pour sécuriser une liaison dudit au moins un élément de maintien d'élément d'appui (56, 58) avec le premier et/ou le deuxième élément d'appui d'implant probatoire (78) dans l'une des positions angulaires.

10. Instrument selon l'une des revendications précédentes, **caractérisé en ce que** la fente comprend une rainure annulaire (88) s'étendant dans la direction périphérique autour de la protubérance (86), et **en ce que** l'élément de coincement est un anneau élastique (90) dont le diamètre est plus grand qu'une profondeur de la rainure annulaire (88).

11. Instrument selon l'une des revendications précédentes, **caractérisé en ce qu'**il est prévu un dispositif de serrage pour sécuriser par serrage une liaison dudit au moins un élément de maintien d'élément d'appui (56, 58) avec le premier et/ou le deuxième élément d'appui d'implant probatoire (78) dans l'une des positions angulaires.

12. Instrument selon l'une des revendications précédentes, **caractérisé en ce qu'**il est prévu une pluralité d'implants probatoires (76), et **en ce que** la pluralité d'implants probatoires (78) présentent des distances d'espacement différentes des surfaces d'appui (80) et/ou des angles de lordose (98) différents entre les surfaces d'appui (80).

13. Instrument selon l'une des revendications précédentes, **caractérisé en ce qu'**il est prévu un dispositif de réglage d'angle (66, 68, 70, 72, 92) pour prédéterminer et régler les positions angulaires différentes du premier et/ou du deuxième élément d'appui d'implant probatoire (78) par rapport audit au moins un élément de maintien d'élément d'appui (56, 58), **en ce que** le dispositif de réglage d'angle (66, 68, 70, 72, 92) comprend au moins deux éléments de réglage (66, 68, 70, 72, 92) pouvant être amenés en prise réciproque dans les différentes positions angulaires, et **en ce que** le premier et/ou le deuxième élément d'appui d'implant probatoire (78) présente un premier élément de réglage (92), et ledit au moins un élément de maintien d'élément d'appui (56, 58) présente un deuxième élément de réglage (66, 68, 70, 72).

14. Instrument selon la revendication 13, **caractérisé en ce qu'**au moins un desdits au moins deux éléments de réglage (66, 68, 70, 72, 92) est réalisé sous la forme d'un logement de réception de réglage (66, 68, 70, 72), et **en ce qu'**au moins un autre desdits au moins deux éléments de réglage (66, 68, 70, 72, 92) est réalisé sous la forme d'une protubérance de réglage (92) pouvant être introduite dans le logement de réception de réglage (66, 68, 70, 72).

15. Instrument selon la revendication 14, **caractérisé en ce que** la protubérance de réglage (92) peut être introduite dans le logement de réception de réglage (66, 68, 70, 72), dans une direction (64) transversale à la direction longitudinale (44).

16. Instrument selon l'une des revendications 14 ou 15, **caractérisé en ce que** l'élément d'appui d'implant probatoire (78) porte au moins une protubérance de réglage (92), et **en ce que** l'élément de maintien d'élément d'appui (56, 58) présente au moins deux logements de réception de réglage (66, 68, 70, 72).

17. Instrument selon l'une des revendications précédentes, **caractérisé en ce que** ledit au moins un élément de maintien d'élément d'appui (56, 58) est de forme cylindrique, et **en ce qu'**un axe longitudinal (64) dudit au moins un élément de maintien d'élément d'appui (56, 58) s'étend transversalement à la direction longitudinale (44).

18. Instrument selon l'une des revendications précédentes, **caractérisé en ce que** les éléments d'appui d'implant probatoire (78) présentent chacun un évidement pour ledit au moins un élément de maintien d'élément d'appui (56, 58), et **en ce que** ledit au moins un élément de maintien d'élément d'appui (56, 58) peut être introduit par complémentarité de formes ou sensiblement par complémentarité de formes, dans l'évidement (84).

19. Instrument selon l'une des revendications précédentes, **caractérisé en ce que** le premier et/ou le deuxième élément d'appui d'implant probatoire (78) peut, dans la deuxième position angulaire par rapport au dit au moins un élément de maintien d'élément d'appui (56, 58), être agencé de manière à être tourné d'un angle de réglage (74) par rapport à la première position angulaire.

20. Instrument selon la revendication 19, **caractérisé en ce que** l'angle de réglage (74) présente une valeur située dans une plage de 10° à 30°.

21. Instrument selon l'une des revendications précédentes, **caractérisé en ce qu'**à l'extrémité distale de l'instrument (10) sont prévu un premier et un deuxième élément de maintien d'élément d'appui (56, 58), et **en ce que** le premier élément de maintien d'élément d'appui (56) peut être relié de manière amovible avec le premier l'élément d'appui d'implant probatoire (78), et le deuxième élément de maintien d'élément d'appui (58) avec le deuxième l'élément d'appui d'implant probatoire (78).

22. Instrument selon la revendication 21, **caractérisé en ce que** le premier élément de maintien d'élément d'appui (56) et le deuxième élément de maintien d'élément d'appui (58) sont montés de manière à pouvoir être rapprochés l'un de l'autre et/ou écartés l'un de l'autre, dans une direction transversale ou sensiblement transversale à leurs surfaces d'appui (80).

23. Instrument selon l'une des revendications 21 ou 22, **caractérisé en ce que** l'extrémité distale de la pièce de maintien (28) présente deux mâchoires d'écartement (32, 34), **en ce que** les mâchoires d'écartement (32, 34) enserrent ou portent les éléments de maintien d'élément d'appui (56, 58), et **en ce que** les mâchoires d'écartement (32, 34) peuvent être écartées l'une de l'autre et/ou rapprochées l'une de l'autre grâce à un dispositif d'entraînement agencé sur la pièce de maintien (28) et comprenant un élément d'actionnement agencé à l'extrémité proximale de la pièce de maintien (28).

24. Instrument selon la revendication 23, **caractérisé en ce que** les mâchoires d'écartement (32, 34) sont montées de manière à pouvoir être écartées parallèlement l'une à l'autre.

25. Instrument selon l'une des revendications 22 à 24, **caractérisé en ce que** la pièce de maintien (28) comporte un dispositif de mesure de déplacement pour mesurer et afficher une distance d'espacement entre les surfaces d'appui (80), ou une valeur de déplacement des éléments de maintien d'élément d'appui (56, 58) l'un par rapport à l'autre.
